(19) Europäisches Patentamt
European Patent Office
Office européen des brevets

(11) Numéro de publication: **0 242 289 B1**

(12) **FASCICULE DE BREVET EUROPEEN**

(45) Date de publication de fascicule du brevet: **19.08.92**

(51) Int. Cl.[5]: **C07D 513/04**, //A61K31/54, (C07D513/04,279:00,265:00)

(21) Numéro de dépôt: **87400833.7**

(22) Date de dépôt: **13.04.87**

Le dossier contient des informations techniques présentées postérieurement au dépôt de la demande et ne figurant pas dans le présent fascicule.

# (54) Procédé de préparation de dérivés d'oxazinobenzothiazine 6,6-dioxyde.

(30) Priorité: **15.04.86 FR 8605377**

(43) Date de publication de la demande: **21.10.87 Bulletin 87/43**

(45) Mention de la délivrance du brevet: **19.08.92 Bulletin 92/34**

(84) Etats contractants désignés: **AT BE CH DE ES FR GB GR IT LI LU NL SE**

(56) Documents cités: **GB-A- 2 160 523**

(73) Titulaire: **LABORATORIOS DEL DR. ESTEVE, S.A.**
**Av. Mare de Deu de Montserrat, 221**
**E-08026 Barcelona(ES)**

(72) Inventeur: **Frigola Constansa, Jordi**
**Av. Diagonal, 299 at. 1a**
**E-08013 Barcelone(ES)**
Inventeur: **Colombo Pinol, Augusto**
**Av. Chile, 36, 4o 1a**
**E-08032 Barcelone(ES)**
Inventeur: **Pares Corominas, Juan**
**Padilla, 349, 3o 3a**
**E-08025 Barcelone(ES)**

(74) Mandataire: **Martin, Jean-Jacques et al**
**Cabinet REGIMBEAU 26, Avenue Kléber**
**F-75116 Paris(FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

EP 0 242 289 B1

## Description

La présente invention concerne un procédé pour la préparation de dérivés de 5 méthyl-3-hétéroaryl-2H, 5H-1, 3-oxazino [5,6-c] [1,2]-benzothiazine-2,4-(3H)-dione 6,6-dioxydes, répondant à la formule générale I ci-dessous, dotés d'un excellent rendement et conduisant à des dérivés très purs

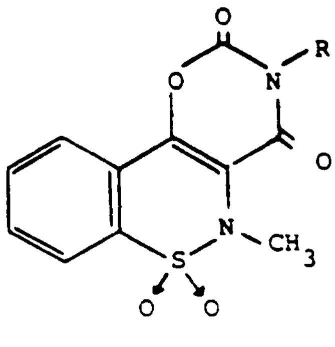

(I)

Les oxazinobenzothiazines 6,6-dioxyde, de formule générale I, dans laquelle R représente un radical hétéroaryle, de préférence pyrimidinyle ou pyridyle substitué ou non substitué, sont des agents anti-inflammatoires et analgésiques bien connus, particulièrement le droxicam, décrits dans Chem. Abst.,1984,100,191893j.

Dans la technique antérieure précédemment citée, on préparait les oxazinobenzothiazines 6,6-dioxyde à partir d'un ester de formule générale II :

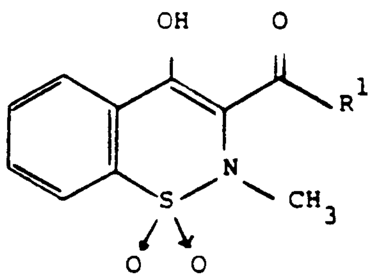

(II)

dans laquelle $R^1$ représente un radical alcoxy, tel que méthoxy ou éthoxy.

En outre, FR-A-2 566 408 décrit la préparation de 5-méthyl-3-(2-pyridyl)-2H,5H-1,3-oxazino [5,6-c] 1,2-benzothiazine-2,4-(3H)-dione 6,6-dioxyde à partir d'un composé de formule II, dans laquelle $R^1$ représente le radical 2-pyridylamino, par réaction avec du phosgène en présence d'un solvant organique inerte, vis-à-vis de la réaction et d'un accepteur d'acide. Pour des raisons d'économie du procédé et pour obtenir le meilleur rendement, le solvant organique est de préférence constitué par le chlorure de méthylène, et la triéthylamine est utilisée en tant qu'accepteur d'acide. Un tel procédé présente cependant de graves inconvénients surtout dans l'hypothèse d'une mise en oeuvre industrielle. Il implique tout d'abord l'utilisation de phosgène qui est un réactif très toxique, et il conduit à un rendement beaucoup trop faible (56%).

La présente invention concerne un nouveau procédé pour la préparation des dérivés de formule générale I précédemment définie, qui permet précisément de résoudre les inconvénients précités. Conformément à l'invention, on prépare les dérivés de formule générale I en faisant réagir un amide de formule générale II, dans laquelle $R^1$ représente un radical hétéroarylamino, de préférence pyrimidinylamino ou pyridylamino substitué ou non substitué, avec un composé de formule générale III :

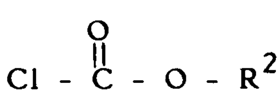

(III)

dans laquelle $R^2$ représente un radical alkyle inférieur en $C_1$ à $C_4$ tel que méthyle ou éthyle, un radical

aryle tel que phényle ou un radical alkylaryle tel que benzyle.

La réaction entre les composés de formules générales II et III s'effectue à des températures comprises entre environ -5° et environ 50°C pendant un temps sensiblement compris entre 3 Heures et 48 heures.

Selon la caractéristique essentielle du procédé de la présente invention, la réaction entre ces deux composés s'effectue au sein d''un solvant organique choisi parmi la pyridine, les pyridines substituées telles que la 4-diméthylaminopyridine, la 4-(1-pyrrolidinyl)-pyridine, la 2,6-ditert-butyl-4-méthylpyridine ainsi que leur mélange. Lorsuqe l'on fait appel à un mélange de pyridine et de pyridine substituée, ces dernières seront utilisées à raison d'une faible proportion pondérale, par exemple de 1 à 10%.

En opérant de la sorte, on obtient, conformément à la présente invention des dérivés de formule générale I ayant un degré de pureté très élevé. Ces dérivés sont en outre obtenus par un procédé de mise en oeuvre industrielle très simple et conduisant à un rendement très élévé, supérieur à 90%.

Les exemples 1 à 4 décrivent de façon détaillée, à simple titre d'illustration non limitatif, la préparation de quelques dérivés de formule générale I.

## EXEMPLE 1

Préparation de 5-méthyl-3-(2-pyridyl)-2H, 5H-1,3-oxazino [5,6-c] [1,2]-benzothiazine-2,4-(3H)-dione 6,6-dioxyde

On ajoute goutte à goutte 13,8 litres (144 moles) de chloroformiate d'éthyle à 11,9 kg (36 moles) de N-(2-pyridyl)-4-hydroxy-2-méthyl-2H-1,2-benzothiazine-3-carboxamide 1,1 dioxyde partiellement dissous dans 30 litres de pyridine anhydre, sous agitation et avec réfrigération dans un bain de glace, de façon que la température soit toujours inférieure à 18°C. On agite pendant 24 heures à température ambiante, puis on verse le tout sur 175 litres d'un mélange eau/glace. On filtre et on lave le résidu abondamment avec de l'eau distillée, ensuite avec de l'acide chlorhydrique 0,5 N et finalement une nouvelle fois avec de l'eau distillée. On dissout le résidu dans l'acétone d'où cristallisent 11,62 kg (90,5%) de 5-méthyl-3-(2-pyridyl)-2H,5H-1,3-oxazino [5,6-c] [1,2]-benzothiazine-2,4-(3H)-dione 6,6-dioxyde, de point de fusion 264-266°C.
Données spectroscopiques :
I.R. (KBr) : 1185; 1355; 1410; 1640; 1710; 1790 $cm^{-1}$
$^1H$ RMN, $\delta$, [DMSO-$d_6$]: 3,02 (s, 3H); 7,52 (m,2H); 7,92 (m,5H); 8,52 (d,1H).

## EXEMPLE 2

Préparation de 5-méthyl-3-(2-pyrimidinyl)-2H, 5H-1,3-oxazino [5,6-c] [1,2]-benzothiazine-2,4-(3H)-dione 6,6-dioxyde

On ajoute goutte à goutte 13,6g (0,04 moles) de chloroformiate de benzyle au 50% dans le toluène à 3,32g (0,01 mole) de N-(2-pyrimidinyl)-4-hydroxy-2-méthyl-2H-1,2-benzothiazine-3-carboxamide 1,1-dioxyde partiellement dissous dans 25 ml de pyridine anhydre, sous agitation et avec réfrigération dans un bain de glace, de façon que la température reste toujours inférieure à 10°C. On agite pendant 8 heures à 30°C et on verse le tout sur 100 ml d'un mélange eau/glace. On filtre et on lave le résidu abondamment avec de l'eau distillée et assitôt après on agite le résidu dans de l'acétone portée à l'ébullition et on filtre. On obtient 3,3g (92%) de 5-méthyl-3-(26pyrimidinyl)-2H, 5H-1,3-oxazino [5,6-c] [1,2]-benzothiazine-2,4-(3H)-dione 6,6-dioxyde, de point de fusion 282-284°C.
Données spectroscopiques :
I.R. (KBr) : 1180; 1355; 1400; 1632; 1710; 1787 $cm^{-1}$
$^1H$ RMN, ,[DMSO-$d_6$]: 3,05 (s,3H); 7,93 (m,5H); 8,95 (d,2H).

## EXEMPLE 3

Préparation de 5-méthyl-3-[2-(4-méthylpyridyl)]-2H, 5H-1,3-oxazino [5,6-c] [1,2]-benzothiazine-2,4-(3H)-dione 6,6-dioxyde

On ajoute goutte à goutte 5 ml (0,04 mole) de chloroformiate de phényle à 3,45g (0,01 mole) de N-[2-(4-méthylpyridyl)]-4-hydroxy-2-méthyl-2H-1,2-benzothiazine-3-carboxamide 1,1-dioxyde partiellement dissous dans 25 ml de pyridine anhydre, sous agitation à température ambiante. On agite pendant 3 heures à 40°C, on laisse refroidir jusqu'à température ambiante, puis on verse le tout sur 100 ml d'un mélange eau/glace. On filtre et on lave abondamment le résidu avec de l'eau distillée, ensuite avec de l'acide

chlorhydrique 0,5 N et finalement une nouvelle vois avec de l'eau distillée. On dissout le résidu dans l'acétone d'où cristallisent 3,1g (83%) de 5-méthyl-3-[2-(4-méthyl-pyridyl)]-2H, 5H-1,3-oxazino [5,6-c] [1,2]-benzothiazine-2,4-(3H)-dione 6,6-dioxyde, de point de fusion 259-261 ° C.
Données spectroscopiques :
I.R. (KBr) : 1195; 1360; 1415; 1640; 1720; 1795 $cm^{-1}$
$^1H$ RMN, $\delta$, [DMSO-$d_6$]: 2,42 (s, 3H); 3,10 (s, 3H); 7,40 (m, 2H); 7,98 (m, 4H); 8,40 m(d, 1H).

EXEMPLE 4

Préparation de 5-méthyl-3-[2-(6-méthylpyridyl)]-2H, 5H-1,3-oxazino [5,6-c] [1,2]-benzothiazine-2,4-(3H)-dione 6,6-dioxyde

On ajoute goutte à goutte 3 ml (0,04 mole) de chloroformiate de méthyle à 3,45g (0,01 ml) de N-[2-(6-méthylpyridyl)]-4-hydroxy-2-méthyl-2H-1,2-benzothiazine-3-carboxamide 1,1-dioxyde, partiellement dissous dans 2g de 4-diméthylamino pyridine et 25 ml de pyridine anhydre, sous agitation et avec réfrigération dans un bain de glace, de façon que la température soit toujours inférieure à 10 ° C. On agite pendant 30 heures à température ambiante et on verse le tout sur 100 ml d'un mélange eau/glace. On filtre et on lave le résidu abondamment avec de l'eau distillée, ensuite avec de l'acide chlorhydrique 0,5 N et finalement une nouvelle fois avec de l'eau distillée. On dissout le résidu dans l'acétone d'où cristallisent 3,6g (97%) de 5-méthyl-3-[2-(6-méthylpyridyl)]-2H, 5H-1,3-oxazino [5,6-c] [1,2]-benzothiazine-2,4-(3H)-dione 6,6 dioxyde, de point de fusion 248-250 ° C.
Données spectroscopiques :
I.R. (KBr) : 1190; 1360; 1410; 1640; 1715; 1790 $cm^{-1}$
$^1H$ RMN, $\delta$, [DMSO-$d_6$]: 2,43 (s, 3H); 3,00 (s, 3H); 7,40 (m, 2H); 7,97 (m, 5H).

**Revendications**

1. Procédé de préparation de 5-méthyl-3-hétéroaryl-2H, 5H-1,3-oxazino [5,6-c] [1,2]-benzothiazine-2,4-(3H)-dione 6,6-dioxyde, de formule générale I :

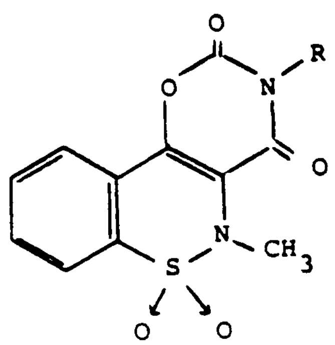

(I)

dans laquelle R représente un radical hétéroaryle, de préférence pyrimidinyle ou pyridyle substitué ou non subsitué, du type consistant à faire réagir un amide de formule générale II :

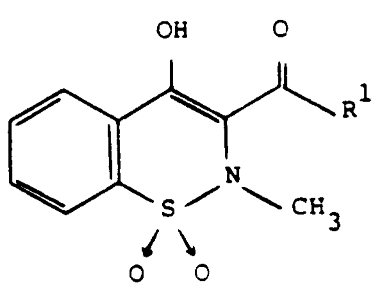

(II)

dans laquelle $R^1$ représente un radical hétéroarylamino, de préférence pyrimidinylamino ou pyridylami-

no substitué ou non subsitué avec un composé de formule générale III :

$$Cl - \overset{\overset{O}{\|}}{C} - O - R^2 \qquad \textbf{(III)}$$

dans laquelle $R^2$ représente un radical alkyle inférieure en $C_1$ à $C_4$ tel que méthyle ou éthyle, un radical aryle tel que phényle ou un radical alkylaryle tel que benzyle, caractérisé en ce que la réaction est effectuée au sein d'un solvant organique choisi parmi la pyridine, les pyridines substituées telles que la 4-diméthylaminopyridine, la 4-(1-pyrrolidinyl)-pyridine, la 2,6-di-tert-butyl-4-méthylpyridine, ainsi que leur mélange.

**2.** Procédé selon la revendication 1, caractérisé en ce que la réaction est effectuée au sein d'un mélange de pyridines contenant 1 à 10% d'au moins une pyridine substituée.

**3.** Procédé selon la revendication 1, caractérisé en ce que la température de réaction est comprise entre environ -5 ° C et environ 50 ° C, pendant une durée sensiblement comprise entre 3 heures et 48 heures.

**4.** Procédé selon l'une des revendications 1 à 3, caractérisé en ce que l'on prépare le 5-méthyl-3-(2-pyridyl)-2H, 5H-1,3-oxazino [5,6-c] [1,2]-benzothiazine-2,4-(3H)-dione 6,6-dioxyde par réaction d'un amide de formule II dans laquelle $R^1$ représente 2-pyridylamino sur un composé de formule III dans laquelle $R^2$ représente le radical éthyle, la réaction étant conduite dans la pyridine, à une température comprise entre -5 ° C et 40 ° C, pendant une durée comprise entre 3 heures et 48 heures.

**5.** Procédé selon l'une des revendications 1 à 3, caractérisé en ce que l'on prépare le 5-méthyl-3-(2-pyrimidinyl)-2H, 5H-1,3-oxazino [5,6-c] [1,2]-benzothiazine-2,4-(3H)-dione 6,6-dioxyde par réaction d'un amide de formule II dans laquelle $R^1$ représente 2-pyrimidinylamino sur un composé de formule III dans laquelle $R^2$ représente le radical benzyle, la réaction étant conduite dans la pyridine, à une température comprise entre -5 ° C et 40 ° C, pendant une durée comprise entre 3 heures et 48 heures.

**6.** Procédé selon l'une des revendications 1 à 3, caractérisé en ce que l'on prépare le 5-méthyl-3-[2-(4-méthylpyridyl)]-2H, 5H-1,3-oxazino [5,6-c] [1,2]-benzothiazine-2,4-(3H)-dione 6,6-dioxyde par réaction d'un amide de formule II dans laquelle $R^1$ représente 2-(4-méthylpyridylamino) sur un composé de formule III dans laquelle $R^2$ représente le radical phényle, la réaction étant conduite dans la pyridine, à une température comprise entre -5 ° C et 40 ° C, penant une durée comprise entre 3 heures et 48 heures.

**7.** Procédé selon l'une des revendications 1 à 3, caractérisé en ce que l'on prépare le 5-méthyl-3-[2-(6-méthylpyridyl)]-2H, 5H-1,3-oxazino [5,6-c] [1,2]-benzothiazine-2,4-(3H)-dione 6,6-dioxyde par réaction d'un amide de formule II dans laquelle $R^1$ représente 2-(6-méthylpyridylamino) avec un composé de formule III dans laquelle $R^2$ représente le radical méthyle, la réaction étant conduite dans la pyridine, à une température comprise entre -5 ° C et 40 ° C, pendant une durée comprise entre 3 heures et 48 heures.

## Claims

**1.** A process for the preparation of a 5-methyl-3-heteroaryl-2H,5H-1,3-oxazino[5,6-c][1,2]benzothiazine-2,4-(3H)-dione 6,6-dioxide of the general formula I:

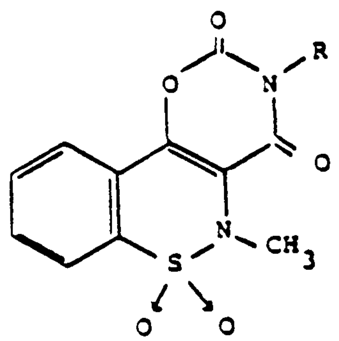

(I)

in which R represents a heteroaryl radical, preferably substituted or unsubstituted pyrimidinyl or pyridyl, of the type consisting in reacting an amide of the general formula II:

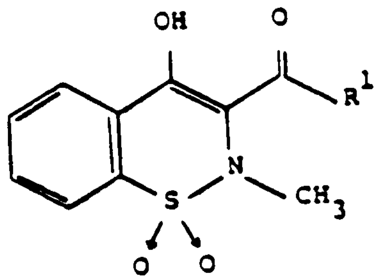

(II)

in which $R^1$ represents a heteroarylamino radical, preferably substituted or unsubstituted pyrimidinylamino or pyridylamino, with a compound of the general formula III:

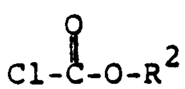

(III)

in which $R^2$ represents a $C_1$ to $C_4$ lower alkyl radical such as methyl or ethyl, an aryl radical such as phenyl, or an alkylaryl radical such as benzyl, wherein the reaction is carried out in an organic solvent selected from the group comprising pyridine, substituted pyridines such as 4-dimethylaminopyridine, 4-(pyrrolidin-1-yl)pyridine and 2,6-ditert.-butyl-4-methylpyridine, and mixtures thereof.

**2.** The process as claimed in claim 1, wherein the reaction is carried out in a mixture of pyridines containing 1 to 10% of at least one substituted pyridine.

**3.** The process as claimed in claim 1, wherein the reaction temperature is between about -5 ° C and about 50 ° C for a period of approximately 3 hours to 48 hours.

**4.** The process as claimed in one of claims 1 to 3, wherein 5-methyl-3-(pyridin-2-yl)-2H,5H-1,3-oxazino-[5,6-c][1,2]benzothiazine-2,4-(3H)-dione 6,6-dioxide is prepared by reacting an amide of the formula II in which $R^1$ represents pyridin-2-ylamino with a compound of the formula III in which $R^2$ represents the ethyl radical, the reaction being carried out in pyridine, at a temperature of between -5 ° C and 40 ° C, for a period of between 3 hours and 48 hours.

**5.** The process as claimed in one of claims 1 to 3, wherein 5-methyl-3-(pyrimidin-2-yl)-2H,5H-1,3-oxazino-[5,6-c][1,2]benzothiazine-2,4-(3H)-dione 6,6-dioxide is prepared by reacting an amide of the formula II in which $R^1$ represents pyrimidin-2-ylamino with a compound of the formula III in which $R^2$ represents the benzyl radical, the reaction being carried out in pyridine, at a temperature of between -5 ° C and 40 ° C, for a period of between 3 hours and 48 hours.

**6.** The process as claimed in one of claims 1 to 3, wherein 5-methyl-3-(4-methylpyridin-2-yl)-2H,5H-1,3-oxazino[5,6-c][1,2]benzothiazine-2,4-(3H)-dione 6,6-dioxide is prepared by reacting an amide of the formula II in which $R^1$ represents 4-methylpyridin-2-ylamino with a compound of the formula III in which $R^2$ represents the phenyl radical, the reaction being carried out in 4-dimethylaminopyridine and pyridine, at a temperature of between -5 ° C and 40 ° C, for a period of between 3 hours and 48 hours.

**7.** The process as claimed in one of claims 1 to 3, wherein 5-methyl-3-(6-methylpyridin-2-yl)-2H,5H-1,3-oxazino[5,6-c][1,2]benzothiazine-2,4-(3H)-dione 6,6-dioxide is prepared by reacting an amide of the formula II in which $R^1$ represents 6-methylpyridin-2-ylamino with a compound of the formula III in which $R^2$ represents the methyl radical, the reaction being carried out in 4-dimethylaminopyridine and pyridine, at a temperature of between -5 ° C and 40 ° C, for a period of between 3 hours and 48 hours.

**Patentansprüche**

**1.** Verfahren zur Herstellung von 5-Methyl-3-heteroaryl-2H,5H-1,3-oxazino[5,6-c]-[1,2]-benzothiazin-2,4-(3H)-dion-6,6-dioxid der allgemeinen Formel I:

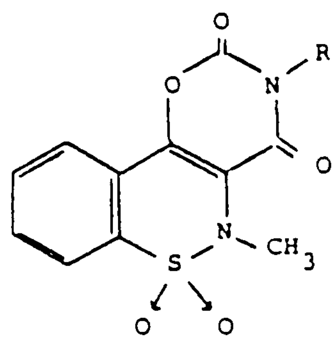

(I)

in der R ein Heteroaryl-Radikal, vorzugsweise ein substituiertes oder nicht substituiertes Pyrimidinyl- oder Pyridyl-Radikal darstellt, des Typs, der darin besteht, ein Amid der allgemeinen Formel II:

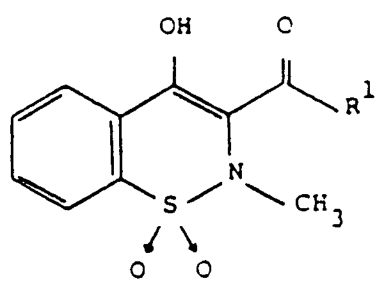

(II)

in der $R^1$ ein Heteroarylamino-Radikal, vorzugsweise ein substituiertes oder nicht substituiertes Pyrimidinylamino- oder Pyridylamino-Radikal darstellt, mit einer Verbindung der allgemeinen Formel III:

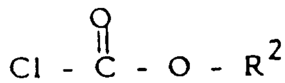

(III)

zur Reaktion zu bringen, in der $R^2$ ein niederes Alkyl-Radikal mit 1 bis 4 Kohlenstoffatomen, wie Methyl oder Ethyl, ein Aryl-Radikal, wie Phenyl oder ein Alkylaryl-Radikal, wie Benzyl darstellt, dadurch gekennzeichnet, daß die Reaktion in einem organischen Lösungsmittel durchgeführt wird, ausgewählt unter Pyridin, den substituierten Pyridinen, wie 4-Dimethylaminopyridin, 4-(1-Pyrrolidinyl)-pyridin, 2,6-Di-tert-butyl-4-methylpyridin sowie deren Mischungen.

2. Verfahren nach Anspruch 1, dadurch gekennzeichnet, daß die Reaktion in einer Pyridin-Mischung durchgeführt wird, die mindestens 1 bis 10 % eines substituierten Pyridins enthält.

3. Verfahren nach Anspruch 1, dadurch gekennzeichnet, daß die Temperatur der Reaktion zwischen etwa -5 °C und etwa 50 °C beträgt, während einer Reaktionsdauer zwischen 3 Stunden und 48 Stunden.

4. Verfahren nach einem der Ansprüche 1 bis 3, dadurch gekennzeichnet, daß man das 5-Methyl-3-(2-pyridyl)-2H,5H-1,3-oxazino[5,6-c]-[1,2]-benzothiazin-2,4-(3H)-dion-6,6-dioxid durch Reaktion eines Amids der Formel II, in der $R^1$ 2-Pyridylamino bedeutet, mit einer Verbindung der Formel III, in der $R^2$ ein Ethyl-Radikal ist, herstellt, wobei die Reaktion in Pyridin bei einer Temperatur zwischen -5 °C und 40 °C während einer Dauer zwischen 3 Stunden und 48 Stunden durchgeführt wird.

5. Verfahren nach einem der Ansprüche 1 bis 3, dadurch gekennzeichnet, daß man das 5-Methyl-3-(2-pyrimidinyl)-2H,5H-1,3-oxazino[5,6-c]-[1,2]-benzothiazin-2,4-(3H)-dion-6,6-dioxid durch Reaktion eines Amids der Formel II, in der $R^1$ 2-Pyrimidinylamino bedeutet, mit einer Verbindung der Formel III, in der $R^2$ ein Benzyl-Radikal ist, herstellt, wobei die Reaktion in Pyridin bei einer Temperatur zwischen -5 °C und 40 °C während einer Dauer zwischen 3 Stunden und 48 Stunden durchgeführt wird.

6. Verfahren nach einem der Ansprüche 1 bis 3, dadurch gekennzeichnet, daß man das 5-Methyl-3-[2-(4-methylpyridyl)]-2H,5H-1,3-oxazino[5,6-c]-[1,2]-benzothiazin-2,4-(3H)-dion-6,6-dioxid durch Reaktion eines Amids der Formel II, in der $R^1$ 2-(4-Methylpyridylamino) bedeutet, mit einer Verbindung der Formel III, in der $R^2$ ein Phenyl-Radikal ist, herstellt, wobei die Reaktion in Pyridin bei einer Temperatur zwischen -5 °C und 40 °C während einer Dauer zwischen 3 Stunden und 48 Stunden durchgeführt wird.

7. Verfahren nach einem der Ansprüche 1 bis 3, dadurch gekennzeichnet, daß man das 5-Methyl-3-[2-(6-methylpyridyl)]-2H,5H-1,3-oxazino[5,6-c]-[1,2]-benzothiazin-2,4-(3H)-dion-6,6-dioxid durch Reaktion eines Amids der Formel II, in der $R^1$ 2-(6-Methylpyridylamino) bedeutet, mit einer Verbindung der Formel III, in der $R^2$ ein Methyl-Radikal ist, herstellt, wobei die Reaktion in Pyridin bei einer Temperatur zwischen -5 °C und 40 °C während einer Dauer zwischen 3 Stunden und 48 Stunden durchgeführt wird.